(19) Europäisches Patentamt
European Patent Office
Office européen des brevets

(11) **EP 0 863 140 B1**

(12) **EUROPÄISCHE PATENTSCHRIFT**

(45) Veröffentlichungstag und Bekanntmachung des Hinweises auf die Patenterteilung:
**02.06.2004   Patentblatt 2004/23**

(51) Int Cl.$^7$: **C07D 295/023**, C07C 209/16, B01J 23/78

(21) Anmeldenummer: **98103813.6**

(22) Anmeldetag: **04.03.1998**

(54) **Verfahren zur N-Alkylierung von Aminen**

Process for N-alkylation of amines

Procédé pour la N-alkylation des amines

(84) Benannte Vertragsstaaten:
**BE CH DE ES FR GB IT LI NL**

(30) Priorität: **07.03.1997   DE 19709488**

(43) Veröffentlichungstag der Anmeldung:
**09.09.1998   Patentblatt 1998/37**

(73) Patentinhaber: **BASF AKTIENGESELLSCHAFT 67056 Ludwigshafen (DE)**

(72) Erfinder:
- **Simon, Joachim, Dr.**
  **68161 Mannheim (DE)**
- **Becker, Rainer, Dr.**
  **67098 Bad Dürkheim (DE)**
- **Lebkücher, Rolf, Dr.**
  **68165 Mannheim (DE)**
- **Neuhauser, Horst, Dr.**
  **67373 Dudenhofen (DE)**

(74) Vertreter: **Isenbruck, Günter, Dr. et al Isenbruck, Bösl, Hörschler, Wichmann, Huhn, Patentanwälte Theodor-Heuss-Anlage 12 68165 Mannheim (DE)**

(56) Entgegenhaltungen:
EP-A- 0 440 829          DE-A- 2 445 303
GB-A- 1 106 084          US-A- 3 709 881

- **CHEMICAL ABSTRACTS, vol. 110, no. 9, 27.Februar 1989 Columbus, Ohio, US; abstract no. 75028p, Seite 603; XP002066169**
- **PATENT ABSTRACTS OF JAPAN vol. 11, no. 163 (C-424), 26.Mai 1987 & JP 61 291551 A (NIPPON NOHYAKU CO LTD), 22.Dezember 1986,**
- **PATENT ABSTRACTS OF JAPAN vol. 14, no. 485 (C-0772), 23.Oktober 1990 & JP 02 202855 A (KAO CORP), 10.August 1990,**
- **PATENT ABSTRACTS OF JAPAN vol. 2, no. 126 (C-025), 21.Oktober 1978 & JP 53 090227 A (MITSUI PETROCHEM IND LTD), 8.August 1978,**

**Beschreibung**

Die vorliegende Erfindung betrifft ein Verfahren zur N-Alkylierung von Aminen, in dem Alkohole mit Alkylaminen oder Dialkylaminen in Gegenwart von Wasserstoff umgesetzt werden.

Nach dem heutigen Stand der Technik werden N-methylsubstituierte Amine üblicherweise hergestellt, indem man Alkohole an hydrierend-dehydrierend wirkenden Katalysatoren auf Basis von Kupfer, Nickel oder Kobalt unter Druck bei erhöhten Temperaturen mit Methylamin oder Dimethylamin umsetzt.

Aus der GB-B-1 106 084 ist die Umsetzung von Diethylenglykol mit Monomethylamin an einem Fällkatalysator aus - beispielsweise - CuO/ZnO bei einer Temperatur im Bereich von 150°C bis 400°C und einem Druck von 30 bis 400 bar bekannt.

Aus der US 3,709,881 ist bekannt, die Reaktion von Diethylenglykol mit Methylamin an einem Nickelkontakt, wie Nickel auf Kieselgur, bei 100 bar und 225 bis 250°C durchzuführen, wobei in 20 bis 60 %iger Ausbeute N-Methylmorpholin erhalten wird.

Alle vorstehenden Verfahren lassen in der Anwendungsbreite und in den erzielten Ausbeuten zu wünschen übrig.

Aus der EP-A-0 440 829 ist ferner bekannt, N-substituierte cyclische Amine aus Diolen und Alkylaminen an einem kupferhaltigen Fällkontakt aus Kupfer auf einem porösen Aluminiumoxid und -oxidhydrat in Gegenwart einer katalytischen Menge von basischen Alkali- oder Erdalkaliverbindungen herzustellen. Obwohl dieses Verfahren technischen Anforderungen in bezug auf die Ausbeute genügen kann, haben die dabei verwendeten Katalysatoren eine eingeschränkte Standzeit und eine unzureichende mechanische Stabilität.

Die in der EP-A-0 440 829 verwendeten Katalysatoren werden gemäß der DE-A-2 445 303 hergestellt, indem man ein Kupfersalz und ein Aluminumsalz, zum Beispiel im Molverhältnis Cu : Al von etwa 0,2 : 1 bis 1 : 1, enthaltende wässrige Lösungen mit einem Alkalimetallcarbonat versetzt und das hierbei anfallende basische Cu/Al-Mischcarbonat zu Katalysatorteilchen wie Kugeln oder Strängen formt und anschließend bei Temperaturen von 200 bis 600 °C trocknet.

Der Erfindung liegt daher die Aufgabe zugrunde, ein technisch und wirtschaftlich vorteilhaftes Verfahren zur N-Alkylierung von Aminen zu finden und den Nachteilen der bekannten Verfahren abzuhelfen.

Diese Aufgabe wird durch ein Verfahren zur N-Alkylierung, in dem Alkohole mit Alkylaminen oder Dialkylaminen in Gegenwart von Wasserstoff umgesetzt werden, wobei die Umsetzung an einem Katalysator auf Basis von Kupfer erfolgt, dadurch gekennzeichnet, dass der Katalysator Magnesiumsilikat und jeweils unabhängig 0,1 bis 2 Gew.-% und BaO, $Cr_2O_3$ und/oder ZnO enthält, gelöst.

Die Alkylreste in den Alkylaminen oder Dialkylaminen weisen dabei unabhängig vorzugsweise 1 bis 6, besonders bevorzugt 1 oder 2 C-Atome auf. Besonders bevorzugt werden Mono- oder Dimethylamin oder Mono- oder Diethylamin eingesetzt.

Die bei dem erfindungsgemäßen Verfahren, das kontinuierlich oder diskontinuierlich - bevorzugt jedoch kontinuierlich - durchgeführt wird, erfolgende Umsetzung wird am Beispiel von Methylamin beziehungsweise Dimethylamin durch die nachfolgend aufgeführte Reaktionsgleichung beschrieben.

$$X\text{-}R\text{-}OH + n \qquad (CH_3)_{3-y}NH_y \rightarrow (CH_3)_{3-y}(H)_{y-1}N\text{-}R\text{-}X$$

oder

$$X = H, OH \qquad [(CH_3)_{3-y}(H)_{y-1}N]_2R \text{ für } X = OH$$

oder

$$y = 1, 2 \qquad CH_3\text{-}N(R\text{-}X)_2$$

oder

$$n = \tfrac{1}{2}, 1, 2 \qquad R\,N\text{-}CH_3 \text{ für } X = OH$$

Das Reaktionsschema gilt auch für die anderen vorstehend aufgeführten Alkylamine beziehungsweise Dialkylamine. Dabei bedeuten im Alkohol R eine Alkylengruppe mit vorzugsweise 1 bis 20, besonders bevorzugt 3 bis 14, insbesondere 4 bis 7, speziell 5 C-Atomen, wobei die Alkylenkette durch 1 oder 2 Sauerstoffatome unterbrochen sein kann, oder insbesondere eine Gruppe -$(CH_2)_p$[-A-$(CH_2)_m$]$_r$ mit A=O, NH oder N-$CH_3$, die gegebenenfalls unabhängig voneinander durch beliebige Reste, wie $C_{1-6}$-Alkylreste oder Halogenatome ein- oder mehrfach substituiert sein können, sowie m,p unabhängig voneinander ganze Zahlen von 2 bis 8 und r 1, 2 oder 3.

Die Alkyl- und Alkylengruppen können verzweigt oder unverzweigt sein. Sie sind vorzugsweise unverzweigt. Die Alkylengruppe ist vorzugsweise eine $\alpha,\omega$-Alkylengruppe. Allgemein eignen sich Alkohole mit primären oder sekundären Hydroxylgruppen für diese Umsetzung, vorzugsweise Alkanole oder Alkandiole, ganz besonders $\alpha,\omega$-Diole. Besonders bevorzugt werden Diethylenglykol oder Pentandiol eingesetzt. Das Molverhältnis von Amin zu Alkohol beträgt bei dem erfindungsgemäßen Verfahren bevorzugt 1:10 bis 3:1, insbesondere 1,2:1 bis 1,8:1.

Gemäß einer bevorzugten Ausführungsform des erfindungsgemäßen Verfahrens wird die Reaktion derart durchgeführt, daß man die Alkohole entweder in flüssiger Phase bei einem Druck von 50 bis 300 bar, bevorzugt 150 bis 250 bar, besonders bevorzugt etwa 200 bar und bei Temperaturen von 150 bis 300°C, bevorzugt 190 bis 250°C, oder in der Gasphase bei einem Druck von 1 bis 40 bar (absolut), bevorzugt 15 bis 35 bar (absolut), und Temperaturen von 150 bis 300 °C, bevorzugt 170 bis 240 °C, mit Mono- oder Dialkylaminen umsetzt.

Der erfindungsgemäß eingesetzte Katalysator basiert auf Kupfer und Magnesiumsilikat. Dabei kann Kupfer in metallischer Form oder als Kupferoxid (CuO) vorliegen. Der Katalysator ist vorzugsweise ein Fällkatalysator beziehungsweise Vollkontakt. Er enthält somit ein Gemisch aus Kupfer beziehungsweise Kupferoxid und Magnesiumsilikat, das insbesondere durch gemeinsame Fällung aus einer kupferhaltigen, magnesiumhaltigen und silikathaltigen Lösung hergestellt wird. Der Gehalt an Kupfer in Form von CuO liegt vorzugsweise im Bereich von 30 bis 60 Gew.-%, besonders bevorzugt 35 bis 50 Gew.-%. Dabei kann das Kupfer beziehungsweise Kupferoxid auch auf Magnesiumsilikat als Träger vorliegen.

Der Katalysator enthält neben Kupfer noch weitere aktivierende Zusätze an Metalloxiden, jeweils unabhängig 0,1 bis 2 Gew.-% BaO, $Cr_2O_3$ und/oder ZnO. Der bevorzugte Gehalt an BaO beträgt 0,5 bis 1,5 Gew.-%. Der Gehalt an $Cr_2O_3$ beträgt vorzugsweise 0,5 bis 1,5 Gew.-%. Der Gehalt an ZnO beträgt vorzugsweise 0,3 bis 1 Gew.-%. Die Gew.-% beziehen sich dabei auf das Gesamtgewicht des fertigen Katalysators. Ferner kann der Katalysator Spuren weiterer Metalle oder Metalloxide enthalten, wie CaO, $Na_2O$ oder $Fe_2O_3$.

Vorzugsweise wird der von der BASF AG, Ludwigshafen, unter der Bezeichnung R3-11 vertriebene Katalysator eingesetzt. Er enthält etwa 45 bis 47 Gew.-% CuO, Magnesiumsilikat aus etwa 15 bis 17 Gew.-% MgO und 35 bis 36 Gew.-% $SiO_2$, etwa 0,9 Gew.-% $Cr_2O_3$, etwa 1 Gew.-% BaO und etwa 0,6 Gew.-% ZnO, sowie Spuren an CaO, $Na_2O$ und $Fe_2O_3$.

Der im erfindungsgemäßen Verfahren eingesetzte Katalysator weist in einer ganz besonders bevorzugten Ausführungsform eine BET-Oberfläche von über 100 $m^2$/g, insbesondere von mehr als 200 $m^2$/g, auf. Die BET-Oberfläche beträgt dabei vorzugsweise 100 bis 400 $m^2$/g, besonders bevorzugt 200 bis 400 $m^2$/g, insbesondere 250 bis 350 $m^2$/g. Dabei ist vorzugsweise die gesamte Oberfläche des Katalysators mit Kupfer oder Kupferoxid bedeckt. Der Katalysator weist vorzugsweise eine Porosität von 0,2 bis 0,7, besonders bevorzugt 0,4 bis 0,6, insbesondere etwa 0,5 ml/g auf.

Der Katalysator, insbesondere der Vollkontakt, ist unter den vorstehend genannten Verfahrensbedingungen mechanisch stabil und zeigt über Wochen keinen merklichen Verlust an Aktivität. Dieses Ergebnis ist insofern überraschend, als bekannt ist, daß feinstverteiltes Kupfer durch Ammoniak und Amine sehr stark angegriffen wird. Die bisher bekannten und für Aminierungen eingesetzten Kontakte enthalten demgemäß - wenn überhaupt - nur relativ wenig Kupfer und weisen wesentlich kleinere BET-Oberflächen auf, die deutlich unter 100 $m^2$/g liegen. So beträgt beispielsweise die BET-Oberfläche des in der EP-A-0 440 829 beschriebenen Katalysators lediglich 83 $m^2$/g.

Der Katalysator wird vorzugsweise als Festbett in Form von Perlen, Pellets, Tabletten, Ringen oder in anderen Formen eingesetzt.

Die Katalysatorbelastung liegt vorzugsweise im Bereich von 0,5 bis 5, besonders bevorzugt 1 bis 3 kg Alkohol pro kg Katalysator (gerechnet als kg Kupfer) und Stunde bei kontinuierlicher Verfahrensführung.

Nach der Umsetzung werden die Produkte durch übliche Verfahren, wie Destillation oder Extraktion, aus dem Reaktionsgemisch isoliert. Nicht umgesetzte Ausgangsstoffe werden gegebenenfalls in die Umsetzung zurückgeführt.

Die Erfindung betrifft auch die Verwendung eines Katalysators, wie er vorstehend beschrieben ist, zur N-Alkylierung von Aminen.

Die folgenden Beispiele dienen der näheren Erläuterung des erfindungsgemäßen Verfahrens. Dabei sind die angegebenen Teile Gewichtsteile.

**BEISPIELE**

**Herstellung des Katalysators**

Die wäßrigen, 20 Gew.-%igen Lösungen von 90 Teilen Magnesiumnitrat, 160 Teilen Kupfer(II)-nitrat und je 2 Teilen Chrom(III)-nitrat, Bariumnitrat und Zinknitrat werden miteinander gemischt und dann bei 20°C unter kräftigem Rühren mit einer wäßrigen, 15 Gew.-%igen Lösung von 220 Teilen Kaliumsilikat versetzt. Die gebildete Suspension wird abgesaugt, und dann wird das Filtergut mit Wasser gewaschen, bis die Waschflüssigkeit keine Nitrationen mehr enthält. Das Filtergut wird bei 40°C 5 Stunden lang vorgetrocknet, dann bei 60°C weitere 5 Stunden lang getrocknet, bei 400°C kalziniert und zu Tabletten verformt. Die reduktive Behandlung wird mit dem einstündigen Erhitzen des Filtergutes im Stickstoffstrom bei 130°C eingeleitet. Anschließend ersetzt man den Stickstoff innerhalb von 2 Stunden nach und nach durch Wasserstoff, wobei die Temperatur auf 220°C angehoben wird. Der Katalysator wird noch weitere 3 Stunden bei 220°C im reinen Wasserstoffstrom gehalten und dann abgekühlt. Der so hergestellte Kontakt hat eine BET-Oberfläche von 290 m$^2$/g, eine Porosität von 0,51 ml/g und eine Schüttdichte von 0,9 kg/l.

### Ausführungsbeispiele

Die Umsetzung wird bei einem Druck von 200 bar in einem kontinuierlich betriebenen Hochdruckreaktor vorgenommen. Der ölbeheizte Reaktor besteht aus V2A-Stahl und hat bei einer Länge von 170 cm einen Innendurchmesser von 3 cm. Während der Umsetzung werden zusätzlich zum Amin und Alkohol noch 300 Nl/h Wasserstoff durch den Reaktor geleitet. Hinter dem Reaktor wird das Produkt in üblicher Weise kondensiert, entspannt und ausgetragen.

### Beispiel 1 - Herstellung von N-Methylmorpholin

Über eine Schüttung von 700 ml des wie vorstehend hergestellten Katalysators in Form von 5 x 3 mm Tabletten werden bei 240°C in Rieselfahrweise stündlich 160 g Monomethylamin und 420 g Diethylenglykol geleitet. Nach einer Laufzeit von 24 Tagen beträgt der Umsatz an Diethylenglykol 98 % und die Selektivität für N-Methylmorpholin liegt bei 77 %.
Der ausgebaute Katalysator zeigt keine mechanischen Veränderungen.

### Vergleichsbeispiel 1

Über eine Schüttung von 700 ml des wie im Herstellungsbeispiel der EP-A 0 440 829 hergestellten Katalysators in Form von 5 x 3 mm Tabletten werden bei 240°C in Rieselfahrweise stündlich 80 g Monomethylamin und 210 g Diethylenglykol geleitet. Nach einer Laufzeit von 13 Tagen beträgt der Umsatz an Diethylenglykol 94 % und die Selektivität für N-Methylmorpholin 64 %.
Der ausgebaute Katalysator ist vollständig zu rotem Schlamm zerfallen.

### Beispiel 2 - Herstellung von N-Methylpiperidin

Über eine Schüttung von 700 ml des wie vorstehend hergestellten Katalysators in Form von 5 x 3 mm Tabletten werden bei 240 °C in Sumpffahrweise stündlich 160 g Monomethylamin und 420 g Pentandiol geleitet. Nach einer Laufzeit von 18 Tagen beträgt der Umsatz an Pentandiol 99 % und die Selektivität für N-Methylpiperidin liegt bei 83 %.
Der ausgebaute Katalysator zeigt keine mechanischen Veränderungen.

### Vergleichsbeispiel 2

Über eine Schüttung von 700 ml des wie im Herstellungsbeispiel der EP-A 0 440 829 hergestellten Katalysators in Form von 5 x 3 mm Tabletten werden bei 240°C in Sumpffahrweise stündlich 160 g Monomethylamin und 420 g Pentandiol geleitet. Nach einer Laufzeit von 16 Tagen beträgt der Umsatz an Pentandiol 99 %, und die Selektivität für N-Methylpiperidin liegt bei 65 %.
Der ausgebaute Katalysator ist vollständig zu rotem Schlamm zerfallen.

### Beispiel 3 - Herstellung von N,N-Dimethylethylamin

Über eine Schüttung von 700 ml des wie vorstehend hergestellten Katalysators in Form von 5 x 3 mm Tabletten werden bei einem Druck von 90 bar und einer Temperatur von 200°C in Sumpffahrweise stündlich 90 g Dimethylamin und 450 g Ethanol geleitet. Nach einer Laufzeit von zwei Tagen beträgt der Umsatz an Dimethylamin > 95 % und die Selektivität für N,N-Dimethylethylamin liegt bei 80 %.
Der ausgebaute Katalysator zeigt keine mechanischen Veränderungen.

**Vergleichsbeispiel 3**

Über eine Schüttung von 700 ml des wie im Herstellungsbeispiel der EP-A 0 440 829 hergestellten Katalysators in Form von 5 x 3 mm Tabletten werden bei einem Druck von 90 bar und einer Temperatur von 200°C in Sumpffahrweise stündlich 90 g Dimethylamin und 450 g Ethanol geleitet. Nach einer Laufzeit von zwei Tagen ist der Umsatz an Dimethylamin vollständig und die Selektivität für N,N-Dimethylethylamin beträgt 90 %.
Der ausgebaute Katalysator ist teilweise zu rotem Schlamm zerfallen.

**Patentansprüche**

1. Verfahren zur N-Alkylierung von Aminen, in dem Alkohole mit Alkylaminen oder Dialkylaminen in Gegenwart von Wasserstoff umgesetzt werden, wobei die Umsetzung an einem Katalysator auf Basis von Kupfer erfolgt, **dadurch gekennzeichnet, dass** der Katalysator Magnesiumsilikat und jeweils unabhängig 0,1 bis 2 Gew.-% BaO, $Cr_2O_3$ und/oder ZnO enthält.

2. Verfahren nach Anspruch 1, wobei der Katalysator 30 bis 60 Gew.-% CuO enthält.

3. Verfahren nach Anspruch 1 oder 2, wobei es sich bei den eingesetzten Aminen um Mono- oder Dimethylamin oder Mono- oder Diethylamin handelt.

4. Verfahren nach einem der Ansprüche 1 bis 3, wobei es sich bei dem eingesetzten Alkoholen um $\alpha,\omega$-Diole handelt.

5. Verfahren nach einem der Ansprüche 1 bis 4, wobei Diethylenglykol mit Monomethylamin und/oder Monoethylamin zu N-Methylmorpholin und/oder N-Ethylmorpholin umgesetzt wird.

6. Verfahren nach einem der Ansprüche 1 bis 4, wobei Pentandiol mit Monomethylamin und/oder Monoethylamin zu N-Methylpiperidin und/oder N-Ethylpiperidin umgesetzt wird.

7. Verfahren nach einem der Ansprüche 1 bis 6, wobei die Umsetzung in flüssiger Phase bei einem Druck von 50 bis 300 bar durchgeführt wird.

8. Verfahren nach einem der Ansprüche 1 bis 6, wobei die Umsetzung in der Gasphase bei einem Druck von 1 bis 40 bar durchgeführt wird.

9. Verfahren nach einem der Ansprüche 1 bis 8, wobei der Katalysator eine Oberfläche nach BET von mindestens 100 $m^2$/g aufweist.

10. Verwendung eines Katalysators, wie er in einem der Ansprüche 1, 2 oder 9 definiert ist, zur N-Alkylierung von Aminen.

**Claims**

1. A process for N-alkylation of amines in which alcohols are reacted with alkylamines or dialkylamines in the presence of hydrogen, the reaction taking place on a catalyst based on copper, wherein the catalyst comprises magnesium silicate and, in each case independently, 0.1 to 2 % by weight of BaO, $Cr_2O_3$ and/or ZnO.

2. A process as claimed in claim 1, where the catalyst comprises 30 to 60 % by weight of CuO.

3. A process as claimed in claim 1 or 2, where the amines employed are mono- or dimethylamine or mono- or diethylamine.

4. A process as claimed in any of claims 1 to 3, where the alcohols employed are $\alpha,\omega$-diols.

5. A process as claimed in any of claims 1 to 4, where diethylene glycol is reacted with monomethylamine and/or monoethylamine to give N-methylmorpholine and/or N-ethylmorpholine.

6. A process as claimed in any of claims 1 to 4, where pentanediol is reacted with monomethylamine and/or monoethylamine to give N-methylpiperidine and/or N-ethylpiperidine.

7. A process as claimed in any of claims 1 to 6, where the reaction is carried out in liquid phase under from 50 to 300 bar.

8. A process as claimed in any of claims 1 to 6, where the reaction is carried out in the gas phase under from 1 to 40 bar.

9. A process as claimed in any of claims 1 to 8, where the catalyst has a BET surface area of at least 100 $m^2$/g.

10. The use of a catalyst as defined in any of claims 1, 2 or 9 for the N-alkylation of amines.


**Revendications**

1. Procédé pour la N-alkylation d'amines, dans lequel on fait réagir des alcools avec des alkylamines ou des dialkylamines en présence d'hydrogène, la réaction se déroulant sur un catalyseur à base de cuivre, **caractérisé en ce que** le catalyseur contient du silicate de magnésium et, à chaque fois indépendamment, 0,1% à 2% en poids de BaO, $Cr_2O_3$ et/ou ZnO.

2. Procédé selon la revendication 1, dans lequel le catalyseur contient 30% à 60% de CuO.

3. Procédé selon la revendication 1 ou 2, dans lequel les amines mises en oeuvre sont une mono- ou une diméthylamine ou une mono-ou une diéthylamine.

4. Procédé selon l'une quelconque des revendications 1 à 3, dans lequel les alcools mis en oeuvre sont des $\alpha,\omega$-diols.

5. Procédé selon l'une quelconque des revendications 1 à 4, dans lequel on fait réagir du diéthylèneglycol avec une monométhylamine et/ou une monoéthylamine pour former de la N-méthylmorpholine et/ou de la N-éthylmorpholine.

6. Procédé selon l'une quelconque des revendications 1 à 4, dans lequel on fait réagir du pentanediol avec une monométhylamine et/ou une monoéthylamine pour former de la N-méthylpipéridine et/ou de la N-éthylpipéridine.

7. Procédé selon l'une quelconque des revendications 1 à 6, dans lequel on réalise la réaction en phase liquide sous une pression allant de 50 bars à 300 bars.

8. Procédé selon l'une quelconque des revendications 1 à 6, dans lequel on réalise la réaction en phase gazeuse sous une pression allant de 1 bar à 40 bars.

9. Procédé selon l'une quelconque des revendications 1 à 8, dans lequel le catalyseur présente une surface spécifique BET de 100 $m^2$/g.

10. Utilisation d'un catalyseur, tel que défini dans l'une quelconque des revendications 1, 2 ou 9, pour la N-alkylation d'amines.